# DEMANDE DE BREVET EUROPEEN

(11) **EP 1 258 947 A1**
(43) Date de publication de la demande: **20.11.2002**
(21) Numéro de dépôt: 01810496.8
(22) Date de dépôt: 17.05.2001
(51) Int. Cl.: H01Q 15/00, A61N 1/16

(54) **Dispositif récepteur-émetteur pour la réduction de champs électromagnétiques environnants**

(71) Demandeur: Rossier, Charles, 1700 Fribourg (CH)
(72) Inventeur: Rossier, Charles, 1700 Fribourg (CH)
(74) Mandataire: Seehof, Michel

(57) **Abrégé**

Le dispositif récepteur-émetteur pour champs électromagnétiques est conçu pour amoindrir les effects des champs électromagnétiques nuisibles. A cet effect il comprend un anneau (2) de préférence non fermé, en matériau conducteur électrique et pourvu d'une connexion capacitive (4) passant par son centre, le diamètre de l'anneau étant égal à la demi-longueur d'onde à neutraliser.
Cet anneau, qui se comporte comme une antenne réceptrice et émettrice, peut amoindrir les effects nuisibles des ondes électromagnétiques et être placé sans ajustement angulaire au croisement des ondes telluriques et radiofréquences, par exemple dans une maison.

## Description

La présente invention se réfère à un dispositif récepteur-émetteur pour champs électromagnétiques, en particulier pour des champs électromagnétiques se trouvant dans la biosphère et perturbant le bien-être notamment de l'homme.

La biosphère dans laquelle nous vivons est baignée en permanence par des champs magnétiques et électriques. La plage de fréquences va du continu aux hyperfréquences. L'origine de cet électromagnétisme peut provenir de deux sources, l'une naturelle, l'autre artificielle.

Depuis environ un siècle, aux ondes électromagnétiques naturelles, qui pratiquement seules baignaient la terre, se sont ajoutées les ondes électromagnétiques artificielles créées par l'homme. Depuis, une quantité croissante, devenue aujourd'hui phénoménale, de sources électriques telles que réseaux de distribution électrique haute-tension, basse-tension, émissions TV, radios, radars, GSM, satellites, etc., polluent l'environnement dans lequel vivent les hommes, les animaux et les végétaux.

Si certaines sources électromagnétiques agressent le corps humain, d'autres en revanche sont bénéfiques pour la vie sur terre.

Le problème consiste donc à amoindrir l'effet des champs électromagnétiques nuisibles au bien-être des personnes et de tous êtres vivants, tout en laissant passer les ondes favorables pour la santé.

Un dispositif connu et en vente est composé d'un tube métallique dont la longueur est calculée en fonction de la longueur d'onde prévue pour l'application. A l'intérieur se trouvent des capacités dont les valeurs sont calculées en fonction de la fréquence des ondes. Mais ce dispositif présente notamment l'inconvénient de devoir être placé dans une direction déterminée pour pouvoir agir.Plus précisement, il convient de le placer exactement dans l'axe de la perturbation, ce qui entraîne un double inconvénient: d'une part, celui de son efficacité, qui est entravée lorsque la pose n'est exacte et, d'autre part, celui du nombre de dispositifs nécessaires.

Le but de la présente invention est de pallier les inconvénients précités en réalisant un dispositif dont l'action de "filtrage" soit optimale et la mise en place aisée. Ce but est atteint grâce aux moyens définis dans la revendication 1.

L'invention sera expliquée en détail ci-après à l'aide des dessins annexés où
- la figure 1: est une vue de dessus du dispositif selon l'invention, et
- la figure 2: montre une coupe selon la ligne II - II dans la figure 1.

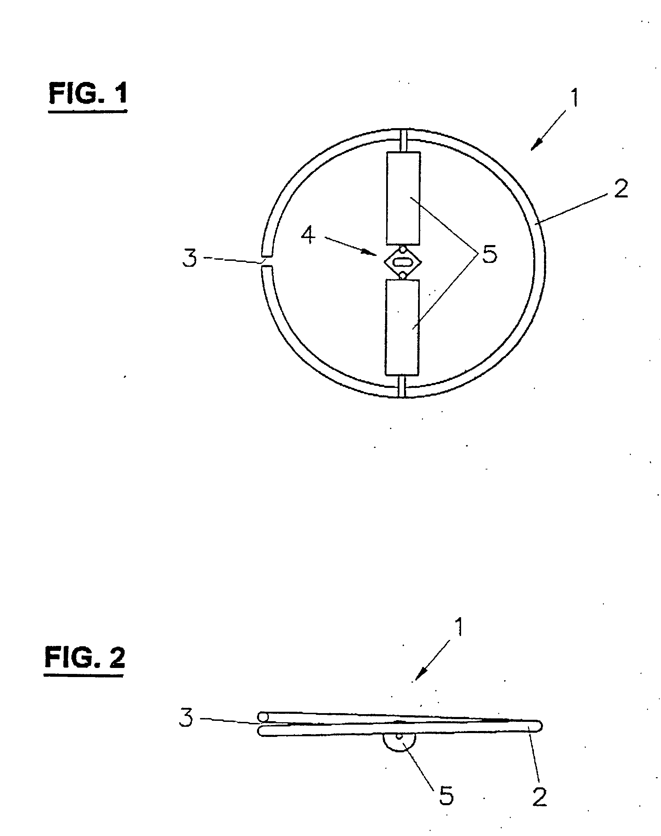

Le dispositif 1 comprend un anneau 2 présentant de préférence une ouverture 3, donc non fermé, et par le centre duquel passe une connexion capacitive 4 composée d'une ou plusieurs capacités 5 (Fig. 1).

Ce dispositif fonctionne à la fois comme antenne absorbatrice et antenne émettrice. Les dimensions de l'anneau, en cuivre ou en tout autre matériau conducteur électrique, et de la connexion capacitive sont choisies en fonction des longueurs d'ondes que l'on veut influencer. De manière avantageuse, les éléments constitutifs du dispositif peuvent être montés sur un circuit imprimé.

Les gammes des ondes (et fréquences)connues aujourd'hui dans le domaine géobiologique sont les suivantes, étant rapellé que le réseau orthogonal, appelé aussi réseau Hartmann, est un réseau tellurique qui parcourt la terre dans la direction Nord-Sud et Est-Ouest, et que le réseau diagonal, appelé aussi réseau de Curry, est également un réseau tellurique, mais qui est orienté Nord-Ouest et Sud-Ouest:

| | Longueur d'onde cm | Fréquence GHz |
|---|---|---|
| Réseaux orthogonal et diagonal | 7.35 à 13.15 | 2.281 à 3.38 |
| Failles et eau | 11.45 à 18.7 | 2.622 à 1.604 |
| Faisceaux hertziens | large plage | large plage |
| Ondes portées sur le réseau électrique | large plage | large plage |

Il est entendu que d'autres fréquences peuvent s'ajouter à cette liste en fonction des découvertes sur ces ondes électromagnétiques.

Dans un premier temps, il appartient au spécialiste de trouver le centre énergétique des rayonnements, c'est-à-dire l'endroit où se croisent les perturbations (notamment telluriques), qu'il s'agit d'éliminer, et les radiofréquences. Cette détermination se fait en général avec un outil permettant de mesurer la fréquence, par exemple avec un appareil appelé antenne Lecher. Les perturbations peuvent se situer, par exemple, à l'intérieur d'une habitation ou d'une voiture, d'un train ou autres moyens de transport.

Dans un second temps, après avoir défini les ondes à supprimer (cf.supra, tableau de valeurs), ledit spécialiste détermine les caractéristiques - diamètre de l'anneau et valeur capacitive - du dispositif le mieux adapté qu'il conviendra de mettre en oeuvre. Le diamètre de l'anneau est choisi avantageusement égal à une demi-longueur d'onde que l'on veut influencer. En pratique, il se situe entre 1 cm et 100 cm, tandis que la valeur capacitive peut s'entendre de 10 pF à 10 µF. Quant à la valeur capacitive, on disposera avantageusement trois éléments capacitifs, soint une capacité centrale et, de part et d'autre de celle-ci, une capacité adjacente (voir figure 1). De manière générale, le diamètre de l'anneau et les capacités sont à adapter aux applications envisagées.

Dans un troisième temps le spécialiste place le dispositif, au centre énergétique et de sorte que les éléments capacitifs soient de préférence orientés Nord-Sud.

A titre d'illustration, lorsque le dispositif est placé à l'intérieur d'une habitation, par exemple un appartement ou une maison, on se réfère aux réseaux orthogonal et diagonal ainsi qu'aux failles et à l'eau, ce qui donne une plage de fréquences comprise entre 1,604 GHz et 3,38 GHz. Le diamètre de l'anneau du dispositif influençant ces ondes sera alors d'environ 65 mm, et la capacité de l'ordre de 120 à 600 pF.

En se référant de façon plus générale au tableau ci-dessus, on peut en déduire que la longueur d'ondes moyenne est de 12 cm. Le diamètre standard de l'antenne du dispositif sera donc de l'ordre de 6 cm. Pour la valeur capacitive, les mesures et essais à l'aide l'antenne Lecher donnent une valeur de 330 pF, une impédance Z de 340 Ω et une résistance Rₛ de 5.7 Ω

A l'intérieur d'une voiture ou d'un train, d'autres fréquences sont à considérer et, de ce fait, un anneau adéquat avec un autre diamètre et une autre connexion capacitive sont à prévoir. Par ailleurs, le dispositif peut être porté sur soi; il peut également traiter des boissons et des aliments.

Le dispositif est avantageusement enrobé dans une matière isolante puis agencé dans une boîte (en un matériau non conducteur électrique, par exemple du bois ou du plastique) ou encore, comme on l'a déjà dit plus haut monté sur une carte de circuit imprimé. L'ensemble peut être fixé à l'endroit choisi dans les conditions exposées plus haut. L'efficacité du dispositif est indépendante de son orientation.

## Revendications

1. Dispositif récepteur-émetteur pour champs électromagnétiques conçu pour amoindrir les effets des champs électromagnétiques nuisibles, **caractérisé en ce qu'**il comprend un anneau (2) en matériau conducteur électrique pourvu d'une connexion capacitive (4) passant par son centre et son diamètre étant approximativement égal à la demi-longueur d'onde de la ou des perturbations à neutraliser.

2. Dispositif selon la revendication 1, **caractérisé en ce que** l'anneau (2) présente une ouverture (3).

3. Dispositif selon la revendication 1 ou 2, **caractérisé en ce que** le diamètre de l'anneau varie entre 1 à 100 cm et que la capacité de la connexion capacitive varie entre 10 pF et 10 µF.

4. Dispositif selon la revendication 1, 2 ou 3, **caractérisé en ce que** la connexion capacitive est constituée de plusieurs capacités (5).

5. Utilisation du dispositif selon l'une des revendications 1 à 4, **caractérisée en ce que** l'on place le dispositif dans le croisement des ondes telluriques et radiofréquences que l'on cherche à amoindrir.
